# EUROPEAN PATENT APPLICATION

(11) **EP 3 091 358 A1**
(43) Date of publication of application: **09.11.2016**
(21) Application number: 15166312.7
(22) Date of filing: 05.05.2015
(51) Int. Cl.: G01N 33/543, G01N 33/551

(54) **METHOD OF REVERSIBLY FUNCTIONALIZING A SOLID SURFACE**

(71) Applicant: Inofea AG, 4057 Basel (CH)
(72) Inventor: Shahgaldian, Patrick, 68300 Saint Louis (FR); Corvini, Philippe, 68220 Leymen (FR); Dudal, Yves, 68220 Hégenheim (FR); Moridi, Negar, 5080 Laufenburg (CH)
(74) Representative: Latscha Schöllhorn Partner AG

(57) **Abstract**

A method of reversibly functionalizing a solid surface (1) comprises the steps of: (i) providing host molecules (2) to the solid surface (1), (ii) covalently immobilizing the host molecules (2) on the solid surface (1), (iii) adding a functional polymer (3) having multiple guest moieties (31) to the solid surface (1), (iv) at least some of the multiple guest moieties (31) of the functional polymer (3) forming supramolecular inclusion complexes with a plurality of the host molecules (2) immobilized on the solid surface (1) such that the functional polymer (3) is bound to the solid surface (1), (v) providing competitor molecules (2) to the functional polymer (3), (vi) the competitor molecules (2) forming supramolecular inclusion complexes with the at least some of the multiple guest moieties (31) of the functional polymer (3), (vii) releasing the functional polymer (3) from the solid surface (1), and (viii) removing the functional polymer (3). The method according to the invention allows for regenerating and changing functionalities of the surface particularly also in place. Thereby the method allows for an efficient reversible modification of more or less any type of surfaces which can be used in a large number of applications.

## Description

### Technical Field

The present invention relates to a method of reversibly functionalizing a solid surface. Such methods supramolecularly modifying a surface can be beneficial in many applications in different fields. For example, such methods can be beneficial for binding an enzyme on the surface of a membrane bioreactor or the like.

### Background Art

For functionalizing surfaces in order to perform specific tasks supramolecular modification of the surfaces often is of fundamental importance. In connection with supramolecular modification of surfaces it has been demonstrated that β-cyclodextrin (β-CD) can form self-assembled monolayers (SAM) on gold surfaces. Synthetic routes have been developed for the synthesis of dialkyl sulphide and alkanethiol derivatives of β-cyclodextrin. Formation of a densely packed SAMs of these synthesized molecules with a hexagonal packing on gold substrate was proved and characterized by atomic force microscopy.

Also, recognition/inclusion properties of β-CD SAMs on gold substrates has been studied and compared with β-CD in solution. Recognition properties of these SAMs for small "guest" molecules such as 1-acetamidoadamantane and ferrocenemethanol were studied and compared with β-CD in solution. It has turned out that their molecular recognition properties were unaltered after immobilization on gold substrates.

Later, a new strategy for reversible supramolecular modification of gold surfaces has been developed. In more details, modified gold substrates with SAMs of β-CDs were used as "host surfaces" for the stable attachment of adamantly-functionalized poly(propylene imine) dendrimers (PPI) as multivalent guest molecules. Stable assemblies were formed on host surfaces due to the formation of multiple, intrinsically weak supramolecular interactions. Desorption of multivalent guest molecules were achieved by introduction of β-CD solution to the system as a competitor host.

In the following, using the same strategy, the ability of immobilized CD-based monolayers on SiO₂ to bind, in a reversible manner, to adamantyl functionalized guest molecules has been demonstrated. The binding properties of the guest molecules to these surfaces, in terms of stability and reversibility, were demonstrated to be similar to that of immobilized β-CD monolayers on gold.

Further, these β-CD modified SiO₂ surfaces have been used as a platform for the precise positioning of multivalent guest molecules. Defined patterns of ferrocenyl (Fc)-functionalized dendrimers (multivalent guest molecules) were transferred onto a β-CD modified substrate using microcontact printing. The stability of the formed complexes was demonstrated in water by solely removing the non-specifically bound molecules during a thorough rinsing of the monolayer. However, local electrochemical conversion of Fc to Fc⁺, achieved using a scanning electrochemical microscopy caused a local release of the surface-bound molecules, owing to the lower affinity of CDs for the ionic ferrocene species.

Still further, a process for patterning these layers via nanoimprint lithography has been described wherein this has been used as a platform for fabrication of 3D nanostructures. First, defined patterns of β-CDs were formed on a silicon oxide surface. Those patterned SAMs were then used as starting points to build three-dimensional objects through a layer-by-layer approach using adamantyl-functionalized dendrimers and β-CD modified nanoparticles (Au 2.8 nm, SiO₂ 60 nm).

Later, protein functionalization of β-CD modified gold surfaces was demonstrated. Ferrocene-tagged yellow fluorescent proteins (Fc-YFPs) were attached in an oriented, reversible fashion to β-CD modified host surfaces. In more details, it was demonstrated that a covalent disulfide bond between two YFP proteins resulted in a switch from monovalent to divalent ferrocene, which resulted in more stable protein immobilization. Fc-YFPs could be patterned on β-CD SAMs as uniform layers. Repetitive adsorption and desorption cycles were interchangeable upon electrochemical reduction and oxidation.

Also, a versatile, orthogonal surface modification method was developed for enzyme immobilization in micro channels. Adamantane-functionalized biomolecules were immobilized on a monolayer of β-CD on gold substrate. More specifically, three building blocks were used in this system: a) an ethylene glycol based mono-adamantyl linker that served for improving the specific protein adsorption; b) a biotinylated bisadamantyl linker for the first multivalent host-guest interactions; c) Streptavidin (SAv) as a second assembly step. Biotinylated calf-intestine alkaline phosphatase was then immobilized onto these SAv modified surfaces. The modification was proved to be specific and reversible. The developed strategy was used for fabrication of enzymatically functionalized microfluidic systems.

Even though the latest approaches turned out to be specific and reversible for many applications they are often not sufficiently efficient implementable and operatable. Therefore, there is a need for a process or system allowing an efficient functionalization of surfaces and an efficient regeneration thereof during operation.

### Disclosure of the Invention

According to the invention this need is settled by a method as it is defined by the features of independent claim 1. Preferred embodiments are subject of the dependent claims.

In particular, the invention deals with a method of reversibly functionalizing a solid surface, comprising the steps of: (i) providing host molecules to the solid surface; (ii) covalently immobilizing the host molecules on the solid surface; (iii) adding a functional polymer having multiple guest moieties to the solid surface; (iv) at least some of the multiple guest moieties of the functional polymer forming supramolecular inclusion complexes with a plurality of the host molecules immobilized on the solid surface such that the functional polymer is bound to the solid surface; (v) providing competitor molecules to the functional polymer; (vi) the competitor molecules forming supramolecular inclusion complexes with the at least some of the multiple guest moieties of the functional polymer; (vii) releasing the functional polymer from the solid surface; and (viii) removing the functional polymer.

It is to be understood that the aforementioned numbering of the steps of the method does not limit the method to a specific order of steps. In particular, the steps of the method according to the invention can as well be executed in a different order than from (i) to (viii) as listed hereinbefore.

In the context of the invention, the term "moiety" can relate to a functional group being a specific group of atoms or bonds within a molecule or at least a part thereof. Such a functional group or a plurality thereof usually is responsible for the characteristic chemical reactions of the respective molecule. The same functional group will undergo the same or similar chemical reactions regardless of the size of the molecule it is a part of.

When being bound to the solid surface, the functional polymer together with the solid surface can form a functional unit. Such a functional unit can be used for breaking down or removing target substances or compounds such as viruses, contaminations, pollutants or the like.

The method according to the invention allows for regenerating and changing functionalities of the surface particularly also in place. This means that the surface can be de- and re-functionalized without requiring removal of the surfaces or the like. This is achieved by using a comparably mild chemical treatment of the surface which is based on supramolecular interactions between the pre-modified surface and the competitor molecules.

Thereby the method allows for an efficient reversible modification of more or less any type of surfaces which can be used in a large number of applications. For example, the method can be used in a bioreactor in which an enzyme is added to a solid surface and replaced when aged. Or, it can be used in connection with the provision of an ion exchange surface, the surface of a biosensor, a lacquer or paint or the like.

Preferably, the host molecules and the competitor molecules are molecules of identical type. This allows for a comparably simple and efficient implementation.

Preferably, the competitor molecules are provided in a solution to the functional polymer and the functional polymer is removed from the solid surface in the solution. Such a provision of the competitor molecules to the surface can allow for completely and rapidly accessing the functional polymer on the surface. Such a provision of the functional polymer from the surface allows for a complete and quick removal of the polymer from the surface. Like this, an efficient provision to and from the surface is possible.

Preferably, the functional polymer is released from the solid surface by its at least some of the multiple guest moieties forming supramolecular inclusion complexes with the competitor molecules. When the functional polymer forms inclusion complexes with competitor molecules the respective guest moieties are no longer bound to molecules fixed to the surface but to freely movable molecules. At a certain point, when the number of connections between the guest moieties and the host molecules is low enough, the functional polymer is no longer held on the surface but released and, e.g., transferred to the solution. This allows for a comparably gentle but still efficient removal of the functional polymer from the surface.

Thereby, the functional polymer is transferred to the solution when being released from the solid surface. Also, the solution preferably is provided to the functional polymer with the competitor molecules at a low concentration when the functional polymer is bound to the solid surface wherein for releasing the functional polymer from the solid surface the concentration of the competitor molecules in the solution is increased to an elevated concentration.

The term "low concentration" in this context can relate to a concentration of the competitor molecules in the solution being too low for releasing the functional polymer from the solid surface. When the competitor molecules are at the low concentration, the functional polymer is in a dynamic equilibrium with regard to the host molecules. Thereby, even though the guest moieties of the functional polymer interact back and forth with the host molecules as well as with the competitor molecules in the dynamic equilibrium state it is bound to the solid surface via the host molecules.

In contrast to the low concentration, the term "elevated concentration" in this context can relate to a concentration of the competitor molecules in the solution being high enough for releasing the functional polymer from the solid surface. In particular, by increasing the concentration of the competitor in the solution an increasing amount of the guest moieties of the functional polymer forms supramolecular complexes with the competitor molecules. At a certain stage the amount of guest moieties of the functional polymer forming supramolecular complexes with the competitor molecules is high enough such that the dynamic equilibrium is disturbed and the functional polymer is no longer bound to the solid surface but transferred to the solution.

Preferably, the functional polymer interacts with a target compound when being bound to the solid surface. Thereby, the functional polymer interacting with the target compound preferably comprises catalyzing a transformation of the target compound. Such catalysis can be performed by a catalytic or enzymatic reaction between the functional polymer and the target compound. Alternatively, the functional polymer interacting with the target compound comprises immobilizing the target compound on the functional polymer. Such interaction allows for immobilizing the target compound and, if desired, to removing it at a certain point. Such method can be used for various different purposes. For example, the method can be used for immobilizing pollutants in water which can be beneficial in waste water treatment. In a regeneration step the solid surface can again be refreshed such the functional polymers bound do no longer carry the pollutions. Similarly, the method can be used for treating air or other media. Like this, efficient applications in various fields can be implemented by means of the method.

Preferably, the functional polymer is covalently attached to an enzyme or to an antibody.

Preferably, the host molecules comprise any of cyclodextrin, calixarene and resorcinarene. Also, the competitor molecules preferably comprise any of cyclodextrin, calixarene and resorcinarene. Such host molecules allow for an efficient implementation of the method suitable for many applications.

Preferably, the functional polymer comprises any of adamantane, ferrocene, cholesterol and steroids. Such functional polymers further allow for an efficient implementation of the method suitable for many applications.

Preferably, the solid surface is comprised in a filtration membrane, a bioreactor surface, an ion exchange surface, a biosensor, a lacquer or a paint.

Preferably, the method further comprises the steps of: adding a further functional polymer having multiple guest moieties to the solid surface after the functional polymer is removed and at least some of the multiple guest moieties of the further functional polymer forming supramolecular inclusion complexes with a plurality of the host molecules immobilized on the solid surface such that the further functional polymer is bound to the solid surface. Like this, the solid surface can be regenerated and reused in further applications. A loss of solid surfaces can be minimized which makes the method particularly cost and resource effective.

These and other aspects of the invention will be apparent from and elucidated with reference to the embodiment(s) described hereinafter.

### Brief Description of the Drawings

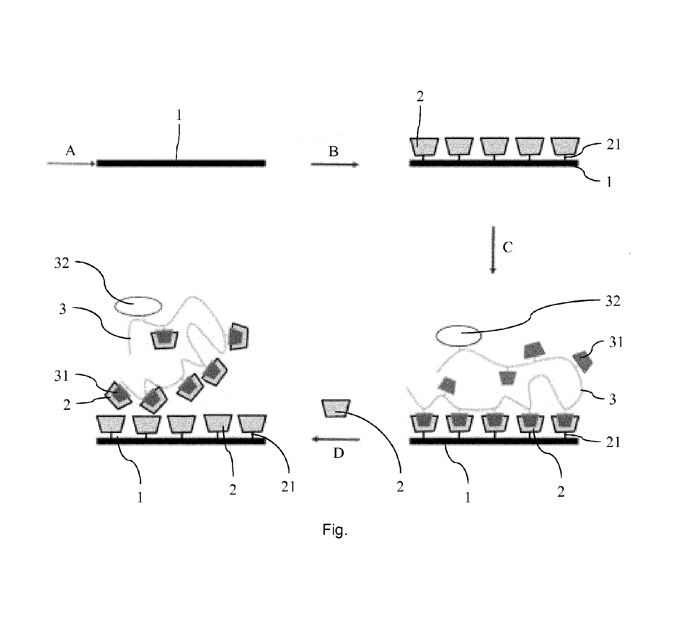

The method according to the invention is described in more detail hereinbelow by way of an exemplary embodiment and with reference to the attached drawing which schematically shows a cycle of the embodiment of the method according to the invention.

### Description of Embodiments

To avoid repetition in the Fig. and the descriptions of the various aspects and illustrative embodiments, it should be understood that many features are common to many aspects and embodiments. Omission of an aspect from a description or Fig. does not imply that the aspect is missing from embodiments that incorporate that aspect. Instead, the aspect may have been omitted for clarity and to avoid prolix description. In this context, the following applies to the rest of this description: If, in order to clarify the drawings, a section of the Fig. contains reference signs which are not explained in the directly associated part of the description, then it is referred to previous or following description sections. Further, for reason of lucidity, if in a section of the Fig. not all features of a part are provided with reference signs it is referred to other sections showing the same part. Like numbers in two or more sections of the Fig. represent the same or similar elements.

The Fig. shows an embodiment of the method according to the invention. Thereby in step A a solid surface, e.g. of a filtration membrane, is provided. In step B cyclodextrin molecules 2 as host molecules are immobilized on the solid surface 1. In particular, the single cyclodextrin molecules 2 are connected to the solid surface 1 by covalent bindings 21.

In step C, an adamantane polymer 3 as functional polymer is added in a solution to the solid surface 1. The adamantane polymer 3 has a plurality of guest moieties 31 and a functional portion 32. The guest moieties 31 interact with the cyclodextrin molecules 2 bound to the solid surface 1. In particular, some of the guest moieties 31 of the adamantane polymer 3 form supramolecular inclusion complexes with the cyclodextrin molecules 2 bound to the solid surface 1. A concentration of the cyclodextrin molecules 2 in the solution as competitor molecules is held in an equilibrium at a low concentration such that a sufficient amount of the guest moieties 31 of the adamantane polymer 3 is connected to the cyclodextrin molecules 2 bound to the solid surface 1. Like this, the adamantane polymer 3 is immobilized to the solid surface 1 and together they form a functional unit.

In this situation, the functional portion 32 of the adamantane polymer 3 can react with a target compound. For example, it can catalytically react with the target compound such that the target compound is transformed and degraded. Or it can bind the target compound to the solid surface 1.

In step D the concentration of cyclodextrin molecules 2 in the solution surrounding the adamantane polymer 3 and the solid surface 1 is raised from the low concentration to an elevated concentration. Thereby, more and more of the guest moieties 31 of the adamantane polymer 3 are interacting with free cyclodextrin molecules 2 which are not bound to the solid surface 1. At a certain concentration the adamantane polymer 3 is released from the solid surface 1, transferred to the solution and can be removed. The solid surface 1 now can be provided with a fresh adamantane polymer 3.

This description and the accompanying drawings that illustrate aspects and embodiments of the present invention should not be taken as limiting the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below. For example, it is possible to operate the invention in various applications such as an enzyme added to a surface of a bioreactor and replaced when aged, an ion exchange surface, a surface of a biosensor, a lacquer or paint or the like.

The disclosure also covers all further features shown in the Fig. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. Method of reversibly functionalizing a solid surface (1), comprising
providing host molecules (2) to the solid surface (1),
covalently immobilizing the host molecules (2) on the solid surface (1), adding a functional polymer (3) having multiple guest moieties (31) to the solid surface (1),
at least some of the multiple guest moieties (31) of the functional polymer (3) forming supramolecular inclusion complexes with a plurality of the host molecules (2) immobilized on the solid surface (1) such that the functional polymer (3) is bound to the solid surface (1),
providing competitor molecules (2) to the functional polymer (3),
the competitor molecules (2) forming supramolecular inclusion complexes with the at least some of the multiple guest moieties (31) of the functional polymer (3),
releasing the functional polymer (3) from the solid surface (1), and
removing the functional polymer (3).

2. Method according to claim 1, wherein the host molecules (2) and the competitor molecules (2) are molecules of identical type.

3. Method according to claim 1 or 2, wherein the competitor molecules (2) are provided in a solution to the functional polymer (3) and the functional polymer (3) is removed from the solid surface (1) in the solution.

4. Method according to any one of the preceding claims, wherein the functional polymer (3) is released from the solid surface (1) by its at least some of the multiple guest moieties (31) forming supramolecular inclusion complexes with the competitor molecules (2).

5. Method according to claim 3 and 4, wherein the functional polymer (3) is transferred to the solution when being released from the solid surface (1).

6. Method according to any one of claims 3 to 5, wherein the solution is provided to the functional polymer (3) with the competitor molecules (2) at a low concentration when the functional polymer (3) is bound to the solid surface (1) wherein for releasing the functional polymer (3) from the solid surface (1) the concentration of the competitor molecules (2) in the solution is increased to an elevated concentration.

7. Method according to any one of the preceding claims, wherein the functional polymer (3) interacts with a target compound when being bound to the solid surface (1).

8. Method according to claim 7, wherein the functional polymer (3) interacting with the target compound comprises catalyzing a transformation of the target compound.

9. Method according to claim 7, wherein the functional polymer (3) interacting with the target compound comprises immobilizing the target compound on the functional polymer (3).

10. Method according to any one of the preceding claims, wherein the functional polymer (3) is covalently attached to an enzyme or to an antibody.

11. Method according to any one of the preceding claims, wherein the host molecules (2) comprise any of cyclodextrin, calixarene and resorcinarene.

12. Method according to any one of the preceding claims, wherein the competitor molecules (2) comprise any of cyclodextrin, calixarene and resorcinarene.

13. Method according to any one of the preceding claims, wherein the functional polymer (3) comprises any of adamantane, ferrocene, cholesterol and steroids.

14. Method according to any one of the preceding claims, wherein the solid surface (1) is comprised in a filtration membrane, a bioreactor surface, an ion exchange surface, a biosensor, a lacquer or a paint.

15. Method according to any one of the preceding claims, further comprising
adding a further functional polymer (3) having multiple guest moieties (31) to the solid surface (1) after the functional polymer (3) is removed and
at least some of the multiple guest moieties (31) of the further functional polymer (3) forming supramolecular inclusion complexes with a plurality of the host molecules (2) immobilized on the solid surface (1) such that the further functional polymer (3) is bound to the solid surface (1).
